# FASCICULE DE BREVET EUROPEEN

(11) **EP 1 205 557 B1**
(45) Date de publication et mention de la délivrance du brevet: **27.06.2007**
(21) Numéro de dépôt: 01402828.6
(22) Date de dépôt: 31.10.2001
(51) Int. Cl.: C12P 7/56

(54) **Procédé de préparation d'un milieu de fermentation à partir d'une matière première renouvelable**
Verfahren zur Herstellung eines Kulturmediums aus erneuerbarem Rohmaterial
Process for the preparation of a fermentation medium from renewable raw material

(30) Priorité: 09.11.2000 FR 0014398
(43) Date de publication de la demande: 15.05.2002
(73) Titulaire: Roquette Frères, 62136 Lestrem (FR)
(72) Inventeur: Caboche, Jean-Jacques, 62131 Drouvin-les-Marais (FR); Duflot, Pierrick, 62136 La Couture (FR); Fouache, Catherine, 62113 Sailly / Labourse (FR); Seigueilha, Laurent, 59130 Lambersart (FR)
(74) Mandataire: Boulinguiez, Didier

(56) Documents cités:
- EP-A- 0 354 828
- EP-A- 1 041 155
- WO-A-94/13826
- WO-A-98/28433
- US-A- 4 698 303
- US-A- 5 681 728
- HONGO M ET AL: "NOVEL METHOD OF LACTIC ACID PRODUCTION BY ELECTRODIALYSIS FERMENTATION" APPLIED AND ENVIRONMENTAL MICROBIOLOGY,US,WASHINGTON,DC, vol. 52, no. 2, août 1986 (1986-08), pages 314-319, XP000852799 ISSN: 0099-2240
- MANNHEIM ADIE ET AL: "Enzyme-modified proteins from corn gluten meal: Preparation and functional properties." JOURNAL OF THE AMERICAN OIL CHEMISTS' SOCIETY, vol. 69, no. 12, 1992, pages 1163-1169, XP002172913 ISSN: 0003-021X

## Description

La présente invention est relative à un procédé particulier de préparation d'un milieu de fermentation à partir d'une matière première renouvelable.

Plus précisément, la présente invention est relative à un procédé particulier de traitement d'une matière première renouvelable, de telle manière qu'il soit possible de l'utiliser directement en fermentation pour la production de métabolites de haute pureté, sans qu'il soit nécessaire de mettre en oeuvre pour les isoler, de nombreuses étapes longues et coûteuses de purification.

Dans la présente invention, on entend par « matière première renouvelable », les déchets des industries alimentaires peu coûteux, non raffinés, généralement non toxiques et riches en sources d'azote et de carbone.

On entend également au sens de l'invention par « métabolites », les produits de transformation par voie fermentaire de sources carbonées directement assimilables par des microorganismes. Il s'agira avantageusement de métabolites choisis dans le groupe constitué par les acides organiques, les vitamines, les acides aminés et les antibiotiques, et préférentiellement les acides organiques comme l'acide L-Lactique.

De manière générale, il est admis que le choix de ladite matière première renouvelable est basé à la fois sur sa disponibilité, sur son coût et sur son aptitude à permettre des productivités élevées.

Il est admis également qu'un milieu de fermentation doit non seulement être constitué par une source carbonée, mais également par une source azotée, auxquelles on ajoute des minéraux et des sels organiques.

La « source carbonée » peut-être tirée de matières premières renouvelables comme les mélasses, les hydrolysats d'amidon de blé, de maïs, de riz, de manioc ou des pommes de terre, mais les « sources carbonées directement assimilables » sont les sucres, raffinés ou purifiés à partir desdites sources carbonées, tels que le glucose, le fructose, le maltose, le saccharose, le lactose et les dextrines.

Des exemples de « sources azotées » ou nutriments protéiques sont quant à eux les extraits de levures, la liqueur de trempe du maïs (encore appelée Corn Steep Liquor), le lait non dénaturé, les protéines des mélasses, les extraits de viande ou la farine de soja. Mais il est souvent préféré d'utiliser les extraits de levures comme sources d'azote et également comme compléments de vitamines et d'éléments minéraux.

Le milieu de fermentation constitué d'une « source carbonée directement assimilable » i.e. glucose ou saccharose, et d'extraits de levures, peut être mis en oeuvre basiquement pour bon nombre de fermentations, tels les fermentations conduisant à la production d'acides organiques, tels les acides lactique, propionique, gluconique, citrique..., les acides aminés essentiels tel la lysine, les antibiotiques ou tout autre métabolite d'intérêt industriel.

Ces milieux conviennent également à la production de biomasse (tel que pour la préparation de ferments lactiques).

Cependant, il est admis que ces milieux présentent l'inconvénient de ne pas être extrapolables à la production à l'échelle industrielle de ces mêmes métabolites d'intérêts (difficulté de disposer de milieux standardisés quant à leur composition et coûts supplémentaires amenés par les étapes de purification ultérieures).

Il est donc choisi, pour baisser les coûts, de mettre en oeuvre des milieux de fermentation où l'une des sources azotées ou hydrocarbonées est apportée par une matière première à bas prix, l'autre composante du milieu de fermentation étant raffinée ou purifiée.

Par exemple, pour la production d'un acide organique comme l'acide lactique :
- dans US 5.416.020 il est décrit un procédé de production d'acide L-Lactique à partir de perméat de petit lait et de petit lait, mais il est encore ajouté de l'extrait de levures en présence de manganèse divalent, avec un mutant de *Lactobacillus delbrueckii* sub. *bulgaricus* ATCC 55163 qui produit essentiellement de l'acide L-Lactique.

Le perméat de petit lait contient bien de 75 à 80 % en poids de lactose, mais ne renferme plus de protéines de grande taille. Il est donc déficient en source d'azote essentielle pour la croissance des microorganismes. D'où le complément rendu nécessaire en extraits de levures. Le petit lait ajouté renferme essentiellement de l'ordre de 65 à 75 % en poids de lactose.

L'extrait de levures fournit alors au milieu de fermentation les nutriments qui ne sont pas apportés de manière adéquate par les perméats de petit lait et le petit lait lui-même.
- Dans US 4.467.034, il est montré qu'il est possible de produire de l'acide lactique à partir du petit lait comme matière première, grâce à un nouveau *Lactobacillus bulgaricus* DSM 2129.

Le petit lait doit cependant encore être complémenté par une source d'azote, i.e. de l'extrait de viande, du corn steep ou de la farine de soja, et également par des vitamines et des sels minéraux.

Dans ces conditions, si la mise en oeuvre de ces milieux de fermentation diminue un peu le coût de la matière première utilisée, elle nécessite de savantes combinaisons respectant le rapport azote / carbone plus l'ajout de compléments nécessaires à une productivité efficace.

En outre, ces milieux « reconstitués » ne sont pas adaptés à la production à haute pureté d'un métabolite comme par exemple l'acide lactique, qui doit en effet être ensuite isolé et purifié par n'importe laquelle des nombreuses techniques conventionnelles, telles que les séparations membranaires, l'échange d'ions, l'extraction par solvants, l'électrodialyse, et la précipitation des sels de lactates.

Il est également décrit dans US 4.769.329 que pour préparer de l'acide lactique optiquement pur avec des *Lactobacillus*, il est nécessaire d'ajouter pour leur croissance un certain nombre de substances qu'elles ne peuvent produire elles-mêmes, par exemple de la biotine, de la thiamine, de l'acide nicotinique, de la pyridoxamine, de l'acide p-aminobenzoïque, de l'acide pantothénique et de la cyanocobalamine.

Or, ces composants doivent être apportés sous la forme de milieux complexes, comme le milieu MRS (développé par de MAN, ROGOSA et SHARPE) mais qui est inutilisable pour la production de l'acide lactique par voie industrielle (coût trop élevé et difficulté également de disposer de milieux standardisés quant à leur composition).

Des milieux complexes du type mélasses de betteraves ou cannes à sucre ou de Corn steep liquor, bien que stimulant la croissance des bactéries, ne peuvent être utilisés pour la préparation d'acide lactique optiquement pur parce qu'ils contiennent eux-mêmes une quantité substantielle d'acide lactique racémique.

De l'acide optiquement pur ne peut donc être obtenu à partir dudit mélange racémique que par des étapes de précipitation et de recristallisation des sels des acides D et L- Lactiques, lourdes et coûteuses.

Pour conduire à l'obtention d'un acide lactique optiquement pur, il est alors recommandé comme dans le procédé de US 4.769.329, d'utiliser comme source de vitamines, azote, sucres et éléments minéraux à l'état de trace, de la levure de boulangerie. Le milieu contient également du glucose, du saccharose ou du lactose comme source carbonée directement assimilable susceptible d'être convertie en acide lactique .

Il est donc nécessaire de revenir à un milieu raffiné et donc très coûteux pour produire un métabolite de haute pureté, ici en l'occurrence de l'acide lactique de haute pureté optiquement actif.

Un certain nombre de solutions ont été proposées pour tenter de remédier aux inconvénients de l'état de la technique précité.

La première solution consiste à utiliser des microorganismes particulièrement résistants à certaines conditions de fermentation ou d'utiliser un cocktail de microorganismes. La seconde, à mettre en oeuvre des techniques de traitement préalable de la matière première renouvelable. Il est également possible de combiner ces deux solutions.

C'est ainsi que par exemple dans US 4.963.486, l'utilisation de maïs comme matière première renouvelable est limitée à son association à un *Rhizopus oryzae* qui a la capacité unique d'amener à la fois les enzymes de saccharification de la matière première et celles permettant la fermentation de celle-ci en acide L-Lactique.

La fermentation s'effectue à une température comprise entre 20 et 40°C, préférentiellement à 30°C. Un agent de neutralisation doit être ajouté pour stabiliser le pH. Le carbonate de calcium est préférentiellement choisi car il présente la particularité de conduire à la formation d'un lactate de calcium qui précipite à 4°C, et permet ainsi la récupération sélective d'un acide lactique de haute pureté.

Le procédé de purification d'acide lactique n'est cependant pas optimisé car il va générer de grandes quantités de gypse préjudiciables pour l'environnement.

Dans US 5.464.760, il est rappelé que la fourniture importante de déchets alimentaires directement fermentescibles et généralement non toxiques donne une source abondante et concentrée de carbone et d'azote pour diverses bactéries aérobies et anaérobies. L'acide lactique peut être alors produit directement à partir de perméat de petit lait, de cannes à sucre ou de betteraves grâce à diverses bactéries lactiques de type *Lactobacillus* avec de haut rendement, par hydrolyse de l'amidon du maïs, de pomme de terre ou de riz, suivi par la bioconversion avec lesdits microorganismes.

Cependant, il est nécessaire d'utiliser des cultures mixtes de cinq souches de bactéries lactiques, et d'effectuer une saccharification et une fermentation.

L'amidon est liquéfié à part, puis replacé dans des conditions de pH et de température particulières permettant d'introduire la glucoamylase avec les bactéries lactiques simultanément.

Mais cette solution n'est pas recommandée, car il est reconnu que l'amidon hydrolysé, tel que les hydrolysats de pomme de terre fabriqués à partir des déchets de pomme de terre ou le sirop de glucose disponible chez tout amidonnier, contiennent normalement jusqu'à 5 % d' « impuretés sucrées » i.e. des pentoses, du maltose et des oligosaccharides, qui restent inutilisées en fin de fermentation ou convertis en d'autres coproduits, tel l'acide acétique, qui provoquent des problèmes pour les étapes de purification subséquentes.

Il est encore nécessaire d'ajouter un complément nutritif à base de sels organiques et minéraux et d'extraits de levures.

Il est même nécessaire de retirer l'acide lactique au fur et à mesure de sa formation dans le milieu de fermentation par électrodialyse continue afin d'éviter de déséquilibrer la population de microorganismes introduite, comme par ailleurs enseigné dans MOTOYOSHI et al dans Appl. Environ. Microbiol. 1986, 52(2), 314-319.

De la même manière, TIWARI et al. dans Zbl. Bakt. II. Abt. Bd., 134, 544-546 (1970) décrivent l'usage de cultures mixtes de *Lactobacillus bulgaricus*, *L. casei* avec ou sans *L. delbrueckii* avec de la mélasse diluée pour la production d'acide lactique.

Cette technique est utilisée pour tenter d'augmenter les rendements de production en acide lactique à partir de mélasses.

Mais le rendement ne dépasse pas au mieux 57,9 %, et les souches interfèrent le plus souvent les unes avec les autres dans leurs capacités de production respectives.

Quant à la deuxième solution qui consiste à effectuer un traitement particulier de la matière première renouvelable, on peut rapporter que dans le brevet US 3.429.777, un des objets de l'invention est d'exploiter la propriété remarquable du lactate de magnésium de cristalliser spontanément à partir du milieu de fermentation renfermant de la mélasse, dans un état de purification suffisant pour permettre la production d'un acide lactique à haut degré de pureté à partir dudit lactate de magnésium.

Le procédé utilisant le magnésium semble donc moins compliqué et moins coûteux que ceux décrits habituellement dans l'état de la technique, comme avec le calcium par exemple. Mais il n'en demeure pas moins vrai que le degré de pureté du lactate de magnésium dans la « liqueur brute sucrée » varie en fonction de la nature et de la qualité de la matière première renouvelable utilisée, même s'il est de meilleure qualité que le lactate de calcium.

La troisième solution consiste enfin à tenir compte à la fois des microorganismes et du traitement de la matière première renouvelable introduite dans le milieu de fermentation.

L'amidon a par exemple été souvent préconisé comme source de carbone bon marché, mais tous les microorganismes ne sont pas capables de le métaboliser, alors que la majorité d'entre eux métabolisent le glucose.

C'est ainsi que dans FR 2.635.534, le procédé décrit propose de conduire la fermentation lactique en présence d'au moins une enzyme amylolytique saccharifiante, mais rien n'est dit sur un moyen d'éliminer les impuretés du milieu de fermentation ainsi traité.

MANHEIM et CHERYAN dans JAOCSS, 69, 12 1992, décrivent l'utilisation contrôlée d'enzymes d'hydrolyse et la technologie des membranes pour isoler des fractions spécifiques du gluten de blé. Ces techniques sont également extrapolables pour le soja.

Pour stimuler l'utilisation en alimentation humaine des protéines de la farine de gluten de blé, cette équipe a développé l'usage des protéases, pour modifier certaines de leurs propriétés fonctionnelles.

Cependant, rien n'est décrit ou ne suggère l'utilisation de ces protéines en industries de fermentation, ni ne décrit ou ne suggère leur utilisation pour la préparation de métabolites aisément purifiables desdits milieux de fermentation.

D'autres stratégies mises en oeuvre ont proposé de ne pas trop modifier le milieu de fermentation et plutôt d'assurer le développement des souches de production, pour accélérer le taux de production microbien et la résistance à de hautes teneurs en acide lactique.

Les outils classiques sont le recyclage de la biomasse, et les cellules immobilisées.

Le recouvrement de l'acide lactique est dans ce cas nécessaire au fur et à mesure de sa production pour éviter son effet inhibiteur de la croissance et de la production bactérienne.

Diverses techniques couplées à la fermentation sont utilisées pour retirer l'acide lactique en continu du milieu de fermentation, i.e. la dialyse, l'électrodialyse, les résines échangeuses d'ions, les bioréacteurs à lit fluidisé bi-particules, l'osmose inverse, et l'extraction liquide-liquide.

Cependant, les coûts du milieu représentent plus de 30 % du coût total de production. De ce fait des nutriments à bas coûts sont indispensables.

Il apparaît donc que de nombreuses tentatives ont été faites pour diminuer les coûts de production de métabolites de haute pureté comme l'acide lactique.

Mais il résulte de tout ce qui précède qu'il existe un besoin non satisfait de disposer d'un procédé simple et efficace permettant de produire par fermentation des métabolites qu'il sera aisé de purifier sans mettre en oeuvre des étapes lourdes, nombreuses et coûteuses, tant au niveau de la préparation du milieu de fermentation, qu'au niveau de la récupération dudit métabolite à partir de ce même milieu de fermentation.

Il est donc nécessaire de mettre au point des conditions de fermentation qui éliminent toutes les impuretés qui habituellement accompagnent la production du métabolite d'intérêt, et surtout compliquent sa purification.

Dans le cas de l'acide lactique, il s'agit des mélanges racémiques de D et L-Lactiques déjà présents dans la matière première renouvelable de départ, et également toutes les « impuretés sucrées » qui ont été évoquées ci-avant et qui encombrent le milieu en fin de fermentation.

Soucieuse de développer un procédé qui permette de répondre mieux que ceux qui existent déjà aux contraintes de la pratique, la société Demanderesse a constaté que cet objectif pouvait être atteint par un procédé consistant à traiter la matière première renouvelable par une combinaison d'étapes enzymatiques afin d'en libérer les sources carbonée et azotée directement assimilables par les microorganismes, et d'étapes particulières de séparation par microfiltration et nanofiltration ou électrodialyse afin d'éliminer du milieu toutes les composantes susceptibles d'altérer la qualité du métabolite à isoler, et qui risquerait de gêner et/ou de compliquer sa purification ultérieure.

Le procédé développé par la société Demanderesse peut alors être avantageusement choisi pour la production de tout métabolite d'intérêt, et préférentiellement des métabolites choisis dans le groupe constitué par les acides organiques, les vitamines, les acides aminés, et les antibiotiques.

Le procédé conforme à l'invention est particulièrement adapté à la préparation d'un métabolite choisi parmi les acides organiques, et est préférentiellement l'acide L-Lactique.

Le procédé développé par la société Demanderesse peut également être choisi pour la production de populations de microorganismes d'intérêt, car il conduit à l'obtention d'un milieu de fermentation débarrassé de toutes impuretés susceptibles de polluer lesdites populations ou de certains inhibiteurs de croissance, comme il sera exemplifié ci-après.

Le procédé de préparation d'un milieu de fermentation permettant la production de métabolites de haute pureté à partir d'une matière première renouvelable conforme à l'invention de la société Demanderesse est caractérisé par le fait qu'il consiste à :
a) éventuellement, traiter ladite matière première renouvelable de manière à l'enrichir en source carbonée ou azotée directement assimilables par des microorganismes, et de manière à en éliminer les impuretés insolubles,
b) éliminer de ladite matière première renouvelable les impuretés de bas poids moléculaires sans altérer son contenu en source carbonée directement assimilable, par une technique choisie dans le groupe constitué de la nanofiltration et de l'électrodialyse, prises seules ou en combinaison,
c) traiter la matière première ainsi débarrassée de ses impuretés de bas poids moléculaire de manière à la compléter en sources azotée ou carbonée directement assimilables par les microorganismes,
d) récupérer le milieu de fermentation ainsi obtenu.

La première étape du procédé conforme à l'invention consiste éventuellement à traiter la matière première renouvelable de manière à l'enrichir en sources carbonée ou azotée directement assimilables par des microorganismes et également de manière à en éliminer les impuretés insolubles.

Ces traitements sont à adapter en fonction de la nature de la matière première renouvelable.

Dans un premier mode de réalisation du procédé conforme à l'invention, on choisit une matière première renouvelable dans le groupe constitué des coproduits d'amidonneries, telles les amidonneries de blé, de maïs, de manioc, de pomme de terre, ou les coproduits de traitement de l'orge, du pois,...

Par exemple, pour la production de l'acide L-Lactique, on choisit avantageusement les coproduits de l'amidonnerie de blé, et plus particulièrement les solubles de blé, ou les coproduits de l'amidonnerie de maïs, et plus particulièrement l'eau de trempe du maïs.

Les matières premières renouvelables contiennent ici à la fois de l'amidon comme source de carbone ou du glucose, et des protéines de haut poids moléculaires, à côté de peptides et acides aminés libres comme source d'azote.

Cependant, s'il est admis que certains microorganismes ont la capacité d'assimiler directement l'amidon ou les protéines de haut poids moléculaire, car ils disposent de l'équipement enzymatique nécessaire à leur dégradation pour leur croissance et pour la production de métabolites d'intérêt, pour d'autres microorganismes, il est nécessaire de les placer dans des conditions où les sources carbonées et azotées sont traitées de manière à être directement assimilables.

Les solubles de blé, par exemple, proviennent du flux de séparation des amidons de blé « B » résultant de la séparation de l'amidon dans le procédé de l'amidonnerie de blé humide. L'amidon B ou amidon second est l'amidon constitué essentiellement par une proportion prépondérante de petits granules d'amidon ou de granules endommagés et contient des impuretés telles que les pentosanes, des protéines et des lipides.

Ces impuretés, dont certaines échappent aux traitements classiques de purification et de déminéralisation, se retrouvent dans les hydrolysats de ces amidons, et rendent ainsi l'amidon B impropre, par exemple, à la fabrication du dextrose de qualité alimentaire. De tels amidons B trouvent ainsi difficilement des débouchés industriels.

La société Demanderesse recommande de les chauffer à une température d'au moins 60°C, et de les traiter au moyen d'une α-amylase et d'une glucoamylase pour en libérer les sucres fermentescibles, et éventuellement au moyen d'une enzyme susceptible de dégrader les fibres végétales, choisie dans le groupe des hémicellulases, pectinases et xylanases comme il sera exemplifié ci-après.

Pour l'eau de trempe du maïs, prélevée directement des silos du trempage de maïs, qui présente une matière sèche comprise entre environ 9 et environ 10 %, ce sont les 35 à 40 % en poids de protéines, composante essentielle de l'eau de trempe, qui présentent l'inconvénient d'être peu assimilables.

La société demanderesse a montré que ces protéines peuvent être traitées en utilisant des enzymes protéolytiques choisies dans le groupe constitué des protéases alcalines, dans des conditions de pH et de température qui permettront de rendre ces protéines plus facilement métabolisables dans l'étape de fermentation qui sera entreprise ultérieurement. Un traitement à pH 7 et à une température de 60°C pendant environ 6 h, à une dose de 1% /sec peut être avantageusement mis en oeuvre.

Dans un second mode de réalisation du procédé conforme à l'invention, on choisit une matière première renouvelable dans le groupe constitué des coproduits résultants du traitement du lait, de l'orge, du soja, de cannes à sucres, de betteraves sucrières, pris seuls ou en combinaison.

Par exemple, pour la production de l'acide L-Lactique, on choisit avantageusement les coproduits résultant du traitement du lait, plus particulièrement du lactosérum et les coproduits résultant du traitement des betteraves sucrières, plus particulièrement des mélasses.

Les matières premières renouvelables utilisées contiennent ici des sources carbonées plus facilement assimilables par la majorité des microorganismes.

C'est ainsi que les mélasses de betteraves renferment essentiellement du saccharose comme source de carbone qui peut être directement assimilée par les microorganismes producteurs d'acide lactique par exemple.

De la même manière, le lactose, composante sucrée essentielle du lactoserum, est facilement assimilé.

Cependant, ce sont les protéines qui sont difficilement assimilables par certains microorganismes.

Dans le cas des coproduits du traitement du lait pour la production d'acide lactique par exemple, il peut être donc avantageusement procédé à une protéolyse de la matière de départ d'origine laitière contenant du lactose avant de faire agir les microorganismes.

Cette étape de protéolyse forme des peptides qui ont un effet activateur vis-à-vis des microorganismes producteurs d'acide lactique.

La matière de départ d'origine laitière contenant du lactose peut-être par exemple un lactosérum doux ou acide, un perméat d'ultrafiltration de lactosérum, du lactose, des eaux mères de cristallisation du lactose, ces matières de départ pouvant contenir, en outre, des protéines sériques ou de la caséine.

Les protéases convenant pour réaliser la protéolyse sont à choisir dans le groupe des pancréatine, trypsine, chymotrypsine, papaïne, ...

En fonction également de la matière première renouvelable choisie, il convient éventuellement ensuite d'éliminer de la dite matière première ainsi enrichie en source carbonée ou azotée directement assimilable les impuretés insolubles de haut poids moléculaire.

Les impuretés insolubles peuvent être majoritairement constituées de fibres.

Par exemple, pour les solubles de blé traités par les enzymes de liquéfaction ou de saccharification de l'amidon, il est avantageusement procédé à la séparation des insolubles par toute technique connue par ailleurs de l'homme du métier, telle la centrifugation et la microfiltration, prises seules ou en combinaison, comme il sera exemplifié ci-après.

La deuxième étape du procédé conforme à l'invention, qui en constitue l'une des caractéristiques essentielles, consiste à traiter la matière première de manière à en éliminer principalement les impuretés de bas poids moléculaires sans altérer son contenu en source carbonée directement assimilable, par une technique choisie dans le groupe constitué de la nanofiltration et de l'électrodialyse, prises seules ou en combinaison.

La société Demanderesse a ainsi vaincu un préjugé technique selon lequel l'utilisation de l'étape de nanofiltration et/ou d'électrodialyse doit s'entendre d'une mise en oeuvre sur le milieu de production d'acide lactique en fin de fermentation, et non pas sur le milieu de fermentation lui-même, avant inoculation par les microorganismes producteurs.

Les milieux de fermentation constitués à partir de matières premières renouvelables renferment un certain nombre « d'impuretés de bas poids moléculaires », i.e. au sens de l'invention, de petites molécules qui gênent les étapes subséquentes de purification des métabolites produits à partir desdits milieux de fermentation.

Ces petites molécules peuvent être par exemple des résidus sucrés qui ne sont pas assimilables par les microorganismes, tels des sucres en C5, qui polluent donc le milieu de fermentation.

Ce peut être également des acides organiques, tel le racémique D et L-Lactique, qui, dans le cas de la production d'acide lactique comme métabolite d'intérêt, empêchent la récupération aisée d'un acide lactique optiquement pur.

Les techniques classiquement mises en oeuvre pour éliminer ces petites molécules sont connues par ailleurs de l'homme du métier, et consistent par exemple en des techniques de filtration sur membrane ou d'électrodialyse conventionnelle adaptées à la gamme de taille desdites impuretés de bas poids moléculaires.

Cependant, ces solutions techniques ne sont pas habituellement retenues par l'homme du métier pour le traitement des milieux de fermentation car les seuils de coupure conduisent également à l'élimination des sources carbonées directement assimilables par les microorganismes, sources carbonées qui présentent une taille en effet située dans la même gamme de taille que ces impuretés.

Toutes ces techniques, comme il a été dit plus haut, sont donc en fait réellement mises en oeuvre sur le milieu mais en fin de fermentation.

La société Demanderesse a donc eu le mérite de montrer, contrairement à tout ce qui était classiquement admis dans l'état de la technique, que ces techniques de filtration sur membrane, et plus particulièrement de nanofiltration, ou d'électrodialyse conventionnelle, permettent d'éliminer les impuretés de bas poids moléculaires et, de manière surprenante et inattendue, sans en altérer le contenu en sources carbonées directement assimilables par les microorganismes.

Les recherches effectuées par la société Demanderesse ont conduit celle-ci à établir les conditions de mise en oeuvre de ces techniques permettant de parvenir au résultat souhaité.

Par exemple, pour la production d'acide lactique à partir de solubles de blé, la société Demanderesse a montré que d'amener la matière sèche du filtrat de microfiltration préparé en suivant les deux premières étapes du procédé conforme à l'invention à une valeur comprise entre 2 et 10 %, de préférence de l'ordre de 4 %, comme il sera exemplifié ci-après pour la séparation par nanofiltration, ou à une valeur comprise entre 5 et 30 %, de préférence de l'ordre de 20 % pour le passage en électrodialyse, permettait de conserver intacte toute la source carbonée, et d'éliminer la quasi totalité du racémique D et L-Lactique.

Quant à la production d'acide lactique à partir d'eau de trempe de maïs traitée par exemple aux protéases alcalines, tel qu'obtenue en suivant l'enseignement des premières étapes du procédé conforme à l'invention, la ramener à une matière sèche comprise entre 1 et 16 %, de préférence de l'ordre de 2 % et la traiter par électrodialyse conventionnelle, comme il sera exemplifié ci-après, conduit également à l'élimination de pratiquement tout le mélange racémique de D et L-Lactique, en ne modifiant pas le contenu en sources carbonées directement assimilables.

La troisième étape du procédé conforme à l'invention consiste à traiter la matière première ainsi débarrassée de ses impuretés de bas poids moléculaire de manière à la compléter en source carbonée ou azotée directement assimilables par les microorganismes

Il peut être en effet choisi, après l'étape de nanofiltration par exemple, de compléter l'apport en sources azotées de la matière première renouvelable ainsi débarrassée de ses impuretés.

Dans le cas des solubles de blé, il est choisi de traiter le rétentat de nanofiltration avec une protéase alcaline de type ALCALASE^{®} de NOVO, comme il sera exemplifié ci-après, afin d'en libérer les peptides.

Dans le cas de l'eau de trempe du maïs, un complément en source carbonée peut être apporté par du glucose, ou par une matière première renouvelable traitée selon le procédé conforme à l'invention.

La dernière étape du procédé conforme à l'invention consiste enfin à récupérer la matière première renouvelable ainsi transformée, et à l'utiliser directement comme milieu de fermentation.

L'enrichissement en source carbonée et azotée directement assimilable, l'élimination des impuretés insolubles et les impuretés de bas poids moléculaire par des techniques peu coûteuses de séparation sur membranes de nanofiltration ou en module d'électrodialyse conventionnelle, permet donc d'obtenir un milieu tout à fait adapté à la production de métabolites d'intérêt, voire à permettre la production de populations de microorganismes débarrassées d'impuretés.

Dans le cas particulier de la production d'acides organiques, et plus particulièrement de l'acide L-Lactique, l'obtention d'un acide lactique optiquement pur, satisfaisant aux normes pharmaceutiques de pureté (test de stabilité thermique de « The United States Pharmacopeia ») et de conformité par rapport aux normes du « Food Chemicals Codex » ne demandera donc plus qu'un nombre limité d'étapes de purification.

D'autres caractéristiques et avantages de l'invention apparaîtront à la lecture des exemples qui suivent. Ils ne sont toutefois donnés ici qu'à titre illustratif et non limitatif.

### Exemple 1

Des solubles de blé à 4 % de matière sèche, provenant du flux de la séparation des amidons de blé « B » sont chauffés à 60°C pendant 15 h et traités au moyen d'une α-amylase TERMAMYL LC de NOVO à raison de 0,05 %/sec, et d'une amyloglucosidase OPTIDEX L 300 A de GENENCOR à raison de 1 %/sec pour libérer les sucres fermentescibles. Les insolubles sont éliminés par microfiltration sur membrane de 0,14 µm.

Le filtrat obtenu, d'une matière sèche de 3,3 %, présente la composition donnée dans le tableau I suivant.

**Tableau I**

| Composants | % en poids/ sec |
|---|---|
| Glucose | 40 |
| Fructose | 10 |
| Hémicellulose | 17 |
| Protéines | 15 |
| Acide D et L-Lactique | 10 |
| Sels et matières grasses | 8 |

Ce filtrat de microfiltration est ensuite nanofiltré sur un module pilote EURODIA de surface 2,5 m², équipé de membranes de nanofiltration DL 2540 à une pression de l'ordre de 20 bars, la température étant régulée à 30°C par refroidissement externe. Le perméat présente une matière sèche de 0,3 % et est principalement composé de 1 g/l d'acide D et L-Lactique, et de l'ordre de 1 g/l de sucres en C5 (xylose et arabinose).

Le rétentat, après concentration par la nanofiltration d'un facteur 4,5, présente une matière sèche de 16 %, et la composition donnée dans le tableau II suivant.

**Tableau II**

| Composants | % en poids/sec |
|---|---|
| Glucose | 43 |
| Fructose | 10 |
| Hémicellulose | 20 |
| Protéines | 18 |
| Acide D et L-Lactique | 2 |
| Sels et matières grasses | 6 |

Cette étape permet ainsi d'éliminer significativement le racémique de D et L-Lactique amené par les solubles de blé.

L'hémicellulose sera éliminée en fin de fermentation avec la biomasse, mais peut être avantageusement hydrolysée, avant l'étape de nanofiltration, à l'aide d'endo et d'exoxylanases connues par ailleurs de l'homme du métier.

On peut effectuer après cette étape de nanofiltration un traitement protéasique dans les conditions suivantes, pour libérer les peptides nécessaires à la constitution de la source azotée directement assimilable du milieu de fermentation.

Aucun ajout de peptides d'origine externe n'est donc ici nécessaire.

Le pH est ajusté à une valeur de 7 et la température amenée à 60°C. On ajoute 1% /sec de protéase AL CALASE^{®} 2.4.L de NOVO et incube à 60°C pendant 4 h.

Après cette étape d'hydrolyse protéasique, le milieu est stérilisé par chauffage 10 min à 120°C et peut être ensuite utilisé directement comme milieu de fermentation.

Le tableau III suivant présente les compositions en acide D et L-Lactique obtenus en fermenteur de 15 l de volume utile avec 13 l de solubles de blé à 16 % de MS traités et non traités.

1,5 l de milieu d'une préculture de 7 h d'une souche de *Lactococcus lactis* sont utilisés pour inoculer ces fermenteurs.

Le pH, fixé à 6,5, est régulé avec NH₄OH 12N. La température est de 40°C.

**Tableau III**

| Nano-Filtration | Traitement ALCALASE^{®} | Durée de la fermentation (h) jusqu'à Glc = 0 | Acide L-Lactique (g/l) | Acide D-Lactique (g/l) |
|---|---|---|---|---|
| Non | Oui | 12 | 59 | 2,8 |
| Oui | Non | 50 | 59 | 0,5 |
| Oui | Oui | 12 | 60 | 0,5 |

La composition finale du milieu de fermentation ne révèle donc que des traces d'acide D-Lactique pour un milieu prétraité par nanofiltration.

Les solubles de blé ainsi traités permettent donc bien d'assurer une fermentation efficace en acide L-Lactique, dépourvue de quantités significatives d'impuretés qui pourraient gêner sa purification ultérieure.

De plus, on constate une bien meilleure productivité lorsque le milieu est prétraité à l'ALCALASE^{®} 2.4.L.

### Exemple 2

Des solubles de blé à 20 % de matière sèche, provenant du flux de la séparation des amidons de blé « B » sont chauffés à 60°C pendant 12 h et traités au moyen d'une α-amylase TERMAMYL LC de NOVO à raison de 0,05 %/sec, d'une amyloglucosidase OPTIDEX L 300 A de GENENCOR à raison de 1% /sec et d'une hémicellulase SPEZYME CP de GENENCOR à raison de 0,5 % /sec, pour libérer les sucres fermentescibles. Les insolubles sont éliminés par microfiltration sur membrane de 0, 14 µm.

Le filtrat obtenu, d'une matière sèche de 16 %, présente la composition donnée dans le tableau IV suivant.

**Tableau IV**

| Composants | % en poids/sec |
|---|---|
| Glucose | 55,4 |
| Fructose | 8,5 |
| Hémicellulose | 11 |
| Protéines | 6,4 |
| Acide D et L-Lactique | 3,8 |
| Sels et matières grasses | 6,3 |

Ce filtrat de microfiltration est ensuite traité par électrodialyse conventionnelle dans un module d'électrodialyse type EUR6B EURODIA, équipé de membranes échangeuses d'ions (NEOSEPTA-TOKUYAMA SODA) de type cationiques CMX-S et anioniques AMX SB de 5,6 m² de surface active en suivant les spécifications du constructeur, ce qui permet d'obtenir une fraction diluée à 13,6 % de matière sèche et présentant la composition donnée par le tableau V suivant.

**Tableau V**

| Composants | % en poids/sec |
|---|---|
| Sucres totaux | 90,5 |
| Protéines hydrolysées | 7,5 |
| Acide D et L-Lactique | 1,5 |
| Sels et divers | 0,5 |

Cette étape permet ainsi d'éliminer significativement le racémique de D et L-Lactique amené par les solubles de blé.

On peut effectuer après cette étape d'électrodialyse un traitement protéasique dans les conditions suivantes, pour libérer les peptides nécessaires à la constitution de la source azotée directement assimilable du milieu de fermentation.

Aucun ajout de peptides d'origine externe n'est donc ici nécessaire. Le pH est ajusté à une valeur de 7 et la température amenée à 60°C. On ajoute 1 % /sec de protéase ALCALASE^{®} 2.4.L. de NOVO et incube à 60°C pendant 4 h.

Après cette étape d'hydrolyse protéasique, le milieu est stérilisé par chauffage 10 min à 120°C et peut être ensuite utilisé directement comme milieu de fermentation.

Le tableau VI suivant présente les compositions en acide D et L-Lactique obtenus en fermenteur de 15 l de volume utile avec 13 l de solubles de blé à 15 % de MS traités et non traités 1,5 1 de milieu d'une préculture de 7 h d'une souche de *Lactococcus lactis* sont utilisés pour inoculer ces fermenteurs. Le pH, fixé à 6,5, est régulé avec NH₄OH 12N. La température est de 40°C.

**Tableau VI**

| Electrodialyse | Traitement ALCALASE^{®} | Durée de la fermentation (h) jusqu'à Glc = 0 | Acide L-Lactique (g/l) | Acide D-Lactique (g/l) |
|---|---|---|---|---|
| Non | Oui | 18 | 80 | 2,2 |
| Oui | Oui | 22 | 80 | 0,8 |

La composition finale du milieu de fermentation ne révèle donc que des traces d'acide D-Lactique pour un milieu prétraité par électrodialyse.

Les solubles de blé ainsi traités permettent donc bien d'assurer une fermentation efficace en acide L-Lactique, dépourvue de quantités significatives d'impuretés qui pourraient gêner sa purification ultérieure.

### Exemple 3

L'eau de trempe prélevée sur un silo intermédiaire de trempage du maïs présente une matière sèche de 3,3 % dont la composition est donnée par le tableau VII suivant.

**Tableau VII**

| Composants | % en poids/sec |
|---|---|
| Sucres totaux | 3 |
| Protéines | 38 |
| Acide D et L-Lactique | 32 |
| Sels et divers | 27 |

Les protéines de maïs étant peu assimilables, on effectue un prétraitement par 1 % /sec d'ALCALASE^{®} de NOVO à pH 7 et 60°C pendant 6 h.

L'hydrolysat ainsi obtenu est ensuite traité par électrodialyse conventionnelle dans un module d'électrodialyse type EUR6B EURODIA, équipé de membranes échangeuses d'ions (NEOSEPTA-TOKUYAMA SODA) de type cationiques CMX-S et anioniques AMX SB de 5,6 m² de surface active en suivant les spécifications du constructeur, ce qui permet d'obtenir une fraction diluée à 2 % de matière sèche et présentant la composition donnée par le tableau VIII suivant.

**Tableau VIII**

| Composants | % en poids/sec |
|---|---|
| Sucres totaux | 4 |
| Protéines hydrolysées | 51 |
| Acide D et L-Lactique | 3 |
| Sels et divers | 42 |

Le prétraitement par l'ALCALASE^{®} et l'élimination d'acides aminés par l'électrodialyse conventionnelle permet de disposer , en début de fermentation, d'un hydrolysat de protéine de maïs de degré d'hydrolyse de 44, pour 36 sur l'eau de trempe brute, tel que déterminé par le rapport azote aminé sur azote total.

Le tableau IX suivant présente les compositions en acide D et L-Lactique obtenus en fermenteur de 15 l de volume utile avec 8,8 l d'eau de trempe de maïs à 2 % de MS traitée et non traitée, auxquels sont ajoutés 60 g/l de glucose comme source de carbone directement assimilable.

1,5 l de milieu d'une préculture de 7 h d'une souche de *Lactococcus lactis* sont utilisés pour inoculer ces fermenteurs. Le pH, fixé à 6,5, est régulé avec du NH₄OH 12N. La température est de 40°C.

**Tableau IX**

| Electrodialyse | Traitement ALCALASE^{®} | Durée de la fermentation (h) jusqu'à Glc = 0 | Acide L-Lactique (g/l) | Acide D-Lactique (g/l) |
|---|---|---|---|---|
| Non | Non | 18 | 62 | 3,3 |
| Oui | Non | 18 | 59 | 0,2 |
| Oui | Oui | 15 | 59 | 0,2 |

L'eau de trempe prétraitée par électrodialyse permet donc bien d'assurer une fermentation efficace en acide lactique, dépourvue ici encore de quantités significatives d'impuretés qui pourraient gêner sa purification ultérieure. On constate ici encore que la productivité est améliorée par prétraitement du milieu de fermentation à l'ALCALASE^{®}.

### Exemple 4

De la mélasse de betteraves concentrée, rediluée à 10 % de matière sèche, est traitée par électrodialyse conventionnelle dans les mêmes conditions que celles de l'exemple 2.

La composition du produit avant électrodialyse est donnée par le tableau X suivant.

**Tableau X**

| Composants | % en poids/sec |
|---|---|
| Sucres (saccharose) | 66 (95) |
| Protéines | 14 |
| Cendres | 12 |
| Acides organiques | 4 |
| Divers (dont bétaïne) | 4 |

Par l'étape d'électrodialyse, on obtient une solution à 5,3 % de matière sèche, sur laquelle la richesse en sucres est de plus de 70 %, et la teneur en protéines de l'ordre de 16 %.

Plus de 90 % des acides organiques, indésirables dans le milieu de fermentation, sont alors éliminés.

### Exemple 5

Dans les mêmes conditions que l'exemple 4, de la mélasse de betteraves concentrée, rediluée cette fois à 20 % de matière sèche, est traitée après microfiltration par électrodialyse conventionnelle dans les mêmes conditions que celles de l'exemple 2.

La composition du produit avant électrodialyse est donnée par le tableau XI suivant.

**Tableau XI**

| Composants | % en poids/sec |
|---|---|
| Sucres (saccharose) | 65,4 (95) |
| Protéines | 11,7 |
| Cendres | 12,3 |
| Acides organiques | 4,4 |
| Divers (dont bétaïne) | 6,2 |

Par l'étape d'électrodialyse, on obtient une solution ici à 16,5 % de matière sèche, sur laquelle la richesse en sucres est de plus de 70 %, et la teneur en protéines de l'ordre de 11 %.

Plus de 90 % des acides organiques, indésirables dans le milieu de fermentation, sont alors éliminés.

On prépare un milieu de fermentation renfermant 80 g/l de mélasse électrodialysée telle que précédemment (milieu B) ou non électrodialysée (milieu A), 5 g/l de (NH4)2SO4, 2 g/l de KH2PO4 et 0,5 g/l de MgSO4.

On ensemence ces milieux de production avec 10 % d'une levure de boulangerie (*S. cerevisiae*), issue d'une préculture de 24 h en milieu glucose 50 g/l et extrait de levure 5 g/l.

Le pH est amené à 5 avec de la NaOH 1N, la température est de 30°C, et la production de biomasse est effectuée dans un réacteur de 15 l de volume utile, en 17 heures sous agitation de 600 rpm et aération de 1 vvm.

Le tableau XII et XIII suivants présentent les résultats obtenus pour la production de S. cerevisiae dans les milieux de production A et B, respectivement.

**Tableau XII**

| | Milieu A | | | | | | |
|---|---|---|---|---|---|---|---|
| Temps | Biomasse | Saccharose | Glc + Fru | EtOH | K | NH4 | PO4 |
| H | g/l | g/l | g/l | g/l | g/l | g/l | g/l |
| 0 | 0,3 | 59 | 3,5 | 1,8 | 4,9 | 1,4 | 2,2 |
| 2 | 0,6 | 54,4 | 3 | 2,3 | nd* | nd | nd |
| 4 | 1 | 52,1 | 4 | 3 | nd | nd | nd |
| 6 | 1,5 | 38,5 | 9,9 | 4,8 | nd | nd | nd |
| 8 | 2,4 | 24 | 15 | 6,9 | nd | nd | nd |
| 14 | 6,8 | 0 | 0 | 18,5 | 6,4 | 0,9 | 2 |
| 17 | 7,4 | 0 | 0 | 16 | nd | nd | nd |

| | | | | | | | |
|---|---|---|---|---|---|---|---|
| * : *nd : non déterminé* | | | | | | | |

**Tableau XIII**

| | Milieu B | | | | | | |
|---|---|---|---|---|---|---|---|
| Temps | Biomasse | Saccharose | Glc + Fru | EtOH | K | NH4 | PO4 |
| H | g/l | g/l | g/l | g/l | g/l | g/l | g/l |
| 0 | 0,3 | 63,2 | 0 | 1,8 | 1,2 | 1,3 | 1,3 |
| 2 | 0,6 | 60,5 | 0 | 2 | nd | nd | nd |
| 4 | 1 | 57,9 | 0 | 2,8 | nd | nd | nd |
| 6 | 1,8 | 46,5 | 4 | 4,4 | nd | nd | nd |
| 8 | 3,1 | 33,7 | 9,9 | 7,1 | nd | nd | nd |
| 14 | 9,6 | 0 | 0 | 18,5 | 4 | 0,5 | 0,8 |
| 17 | 11 | 0 | 0 | 17,5 | nd | nd | nd |

Il s'en déduit que dans un milieu où la mélasse est électrodialysée, le taux de croissance initial est de 0,29 h⁻¹,et le rendement en biomasse est de 24 %, tandis que si on utilise de la mélasse non électrodialysée, on n'obtient qu'un taux de croissance de 0,26 h⁻¹, avec un rendement en biomasse de 17 %.

Il apparaît donc que le traitement de la mélasse selon le procédé conforme à l'invention est tout fait adapté à une production de levures efficace.

### Exemple 6

Du lactosérum est traité par électrodialyse conventionnelle dans les mêmes conditions que celles de l'exemple 2.

La composition initiale du lactosérum de 6,6 % de matière sèche est donnée dans le tableau XIV suivant.

**Tableau XIV**

| Composants | % en poids |
|---|---|
| Sucres | 71 |
| Protéines | 12 |
| Acides organiques | 4 |
| Sels | 9 |
| Matières grasses | 4 |

Par l'étape d'électrodialyse conventionnelle, on obtient une solution de 4,3 % en matière sèche.

La richesse en sucres est supérieure à 80 %, pour une teneur de protéines de l'ordre de 14 %.

La totalité des acides organiques polluants du milieu est ainsi éliminée.

### Exemple 7

Du lactosérum est traité par électrodialyse conventionnelle dans les mêmes conditions que celles de l'exemple 6.

La composition initiale du lactosérum est ici de 16,2 % de matière sèche est donnée dans le tableau XV suivant.

**Tableau XV**

| Composants | % en poids/sec |
|---|---|
| Sucres | 69,2 |
| Protéines | 11 |
| Acides organiques | 3,6 |
| Sels | 14,6 |
| Matières grasses | 1,6 |

Par l'étape d'électrodialyse conventionnelle, on obtient une solution de 14,6 % en matière sèche. La richesse en sucres est supérieure à 80 %, pour une teneur de protéines de l'ordre de 9 %. La totalité des acides organiques polluants du milieu est ainsi éliminée.

Du lactosérum ainsi électrodialysé est incorporé dans un milieu de production de biomasse, en l'occurrence de ferments lactiques (*Streptococcus lactis*) et de levures (*S. cerevisiae*), dans les conditions suivantes.

Un équivalent « öse » de cellules prélevées sur colonies cultivées en boite de Pétri est introduit dans un erlen de 2 1 renfermant 500 ml de milieu de production constitué de 80 g/l de lactosérum électrodialysé ou non (témoin) sous agitation à 150 rpm, et à une température de 30°C pour *S. cerevisiae*, en conditions aérobies et de 40 °C pour *Streptococcus lactis*, en conditions anaérobies.

La richesse en biomasse est établie par le suivi du nombre de cellules.

Le tableau XVI présente les résultats obtenus après culture pendant 24 heures.

**Tableau XVI**

| | *Streptococcus lactis* | *S. cerevisiae* |
|---|---|---|
| Lactosérum microfiltré | 0 | 1,7 10⁸ |
| Lactosérum microfiltré et électrodialysé | 1,7 10⁸ | 2,7 10⁸ |

Le traitement par électrodialyse conforme à l'invention est donc d'une grande efficacité pour la culture des deux souches testées. Dans le cas de levure classique, le milieu de culture électrodialysé permet de doubler pratiquement la biomasse produite, tandis que dans le cas du ferment lactique, le lactosérum microfiltré renferme même un inhibiteur de croissance que l'électrodialyse permet d'éliminer.

## Revendications

1. Procédé de préparation d'un milieu de fermentation permettant la production de métabolites de haute pureté à partir d'une matière première renouvelable, **caractérisé par le fait qu**'il consiste à :
a) éventuellement, traiter ladite matière première renouvelable de manière à l'enrichir en sources carbonée ou azotée directement assimilables par des microorganismes, et de manière à en éliminer les impuretés insolubles,
b) éliminer de ladite matière première renouvelable les impuretés de bas poids moléculaires sans altérer son contenu en source carbonée directement assimilable, par une technique choisie dans le groupe constitué de la nanofiltration et de l'électrodialyse, prise seule ou en combinaison,
c) traiter la matière première ainsi débarrassée de ses impuretés de bas poids moléculaire de manière à la compléter en sources azotée ou carbonée directement assimilables par les microorganismes,
d) récupérer le milieu de fermentation ainsi obtenu.

2. Procédé selon la revendication 1, **caractérisé par le fait que** le métabolite produit par fermentation est choisi dans le groupe constitué par les acides organiques, les vitamines, les acides aminés, et les antibiotiques, et est préférentiellement un acide organique.

3. Procédé selon la revendication 2, **caractérisé par le fait que** l'acide organique est de l'acide L-Lactique.

4. Procédé selon l'une quelconque des revendications 1 à 3, **caractérisé par le fait que** la matière renouvelable est choisie dans le groupe constitué des coproduits d'amidonneries, préférentiellement des coproduits des amidonneries de blé, de maïs, de manioc, de pomme de terre, ou les coproduits de traitement de l'orge, du pois, et est préférentiellement constitué par des solubles de blé ou par de la liqueur d'eau de trempe de maïs.

5. Procédé selon l'une quelconque des revendications 1 à 3, **caractérisé par le fait que** la matière renouvelable est choisie dans le groupe constitué des coproduits résultant du traitement du lait, du soja, de cannes à sucres, de betteraves sucrières, et est préférentiellement constitué par du lactosérum et par des mélasses.

6. Procédé selon l'une quelconque de revendications 1 à 4, **caractérisé par le fait que** l'on enrichit la matière première renouvelable en source carbonée assimilable par des microorganismes en utilisant des enzymes de liquéfaction et de saccharification de l'amidon et éventuellement une enzyme susceptible de dégrader les fibres végétales, choisie dans le groupe des hémicellulases, pectinases et xylanases.

7. Procédé selon l'une quelconque des revendications 1 à 6, **caractérisé par le fait que** l'on enrichit ou complète la matière première renouvelable en source azotée assimilable en utilisant des enzymes protéolytiques choisies dans le groupe constitué des protéases alcalines et des protéases acides.

8. Procédé selon l'une quelconque des revendications 1 à 6, **caractérisée par le fait que** l'on complète la matière première renouvelable en source carbonée assimilable par l'ajout de glucose.

9. Milieu de fermentation susceptible d'être obtenu par un quelconque des procédés des revendications 1 à 8.

10. Utilisation du milieu de fermentation selon la revendication 9 pour la production de populations de microorganismes.

## Claims

1. A process for preparing a fermentation medium for producing high-purity metabolites from a renewable raw material, **characterized by** the fact that it consists of:
a) optionally, treating said renewable raw material to enrich it with carbon or nitrogen sources which can be directly assimilated by micro-organisms and to eliminate insoluble impurities,
b) using a technique chosen from the group consisting of nanofiltration and electrodialysis, alone or in combination, to eliminate low molecular weight impurities from said renewable raw material without degrading its content of carbon sources which can be directly assimilated,
c) treating the raw material from which the low molecular weight impurities have been eliminated in this way to top it up with nitrogen or carbon sources which can be directly assimilated by the micro-organisms, and
d) recovering the resulting fermentation medium.

2. A process according to claim 1, **characterized by** the fact that the metabolite produced by fermentation is chosen from the group consisting of organic acids, vitamins, amino acids, and antibiotics, and is preferably an organic acid.

3. A process according to claim 2, **characterized by** the fact that the organic acid is L-lactic acid.

4. A process according to any of claims 1 to 3, **characterized by** the fact that the renewable material is chosen from the group consisting of by-products of starch manufacture, preferably by-products of manufacture of starch from wheat, corn, cassava, potato, or by-products of processing barley, peas, and preferably consists of wheat solubles or corn steep liquor.

5. A process according to any of claims 1 to 3, **characterized by** the fact that the renewable material is chosen from the group consisting of by-products from processing milk, soya, sugar cane, sugar beet, and preferably consists of lactoserum and molasses.

6. A process according to any of claims 1 to 4, **characterized by** the fact that the renewable raw material is enriched with carbonated sources which can be assimilated by micro-organisms using enzymes for liquefying and saccharifying starch, and optionally an enzyme capable of degrading vegetal fibres, said enzyme being selected from the group consisting of hemicellulases, pectinases and xylanases.

7. A process according to any of claims 1 to 6, **characterized by** the fact that proteolytic enzymes chosen from the group consisting of alkaline proteases and acidic proteases are used to enriched or top up the renewable raw material with nitrogen sources which can be assimilated.

8. A process according to any of claims 1 to 6, **characterized by** the fact that glucose is added to top up the renewable raw material with carbon sources which can be assimilated.

9. A fermentation medium obtainable by a process according to any of claims 1 to 8.

10. Use of a fermentation medium according to claim 9 for the production of populations of microorganisms.

## Patentansprüche

1. Verfahren zur Herstellung eines Kulturmediums, welches die Herstellung von Metaboliten mit hoher Reinheit aus einem erneuerbaren Rohmaterial erlaubt, **dadurch gekennzeichnet, dass** es besteht aus:
a) gegebenenfalls Behandeln des erneuerbaren Rohmaterials auf die Weise, dass es mit durch Mikroorganismen direkt assimilierbaren Kohlenstoff- oder Stickstoffquellen angereichert wird, und auf die Weise, dass unlösliche Verunreinigungen entfernt werden,
b) Entfernen der Verunreinigungen mit niedrigem Molekulargewicht aus dem erneuerbaren Rohmaterial, ohne seinen Gehalt an direkt assimilierbaren Kohlenstoffquellen zu verändern, durch eine Technik ausgewählt aus der Gruppe gebildet aus Nanofiltration und Elektrodialyse, allein oder in Kombination,
c) Behandeln des auf diese Weise von seinen Verunreinigungen mit niedrigem Molekulargewicht befreiten Rohmaterials auf die Weise, dass es mit durch Mikroorganismen direkt assimilierbaren Kohlenstoff- oder Stickstoffquellen ergänzt wird,
d) Gewinnen des auf diese Weise erhaltenen Kulturmediums.

2. Verfahren gemäß Anspruch 1, **dadurch gekennzeichnet, dass** der durch Kultur hergestellte Metabolit aus der Gruppe gebildet aus organischen Säuren, Vitaminen, Aminosäuren, Antibiotika ausgewählt wird, und dass es vorzugsweise eine organische Säure ist.

3. Verfahren gemäß Anspruch 2, **dadurch gekennzeichnet, dass** die organische Säure L-Milchsäure ist.

4. Verfahren gemäß einem der Ansprüche 1 bis 3, **dadurch gekennzeichnet, dass** das erneuerbare Rohmaterial ausgewählt wird aus der Gruppe gebildet aus Koprodukten der Stärkeproduktion, vorzugsweise Koprodukten der Stärkeproduktion aus Weizen, Mais, Maniok, Kartoffel oder den Koprodukten der Behandlung von Gerste, Erbsen, und die Gruppe wird vorzugsweise aus löslichen Stoffen von Weizen oder aus der wässrigen Flüssigkeit von Maismaische gebildet.

5. Verfahren gemäß einem der Ansprüche 1 bis 3, **dadurch gekennzeichnet, dass** der erneuerbare Rohstoff ausgewählt wird aus der Gruppe gebildet aus Koprodukten, welche bei der Behandlung von Milch, Soja, Zuckerrohr, Zuckerrüben entstehen, und die Gruppe wird vorzugsweise aus Molke und aus Melassen gebildet.

6. Verfahren gemäß einem der Ansprüche 1 bis 4, **dadurch gekennzeichnet, dass** das erneuerbare Rohmaterial mit einer durch Mikroorganismen assimilierbaren Kohlenstoffquelle angereichert wird, indem Enzyme zur Verflüssigung und Verzuckerung von Stärke und gegebenenfalls ein Enzym geeignet zum Abbau von Pflanzenfasern ausgewählt aus der Gruppe gebildet aus Hemicellulasen, Pektinasen und Xylanasen verwendet werden.

7. Verfahren gemäß einem der Ansprüche 1 bis 6, **dadurch gekennzeichnet, dass** das erneuerbare Rohmaterial mit einer assimilierbaren Stickstoffquelle angereichert oder ergänzt wird, indem proteolytische Enzyme ausgewählt aus der Gruppe gebildet aus alkalischen Proteasen und sauren Proteasen verwendet werden.

8. Verfahren gemäß einem der Ansprüche 1 bis 6, **dadurch gekennzeichnet, dass** das erneuerbare Rohmaterial mit einer assimilierbaren Kohlenstoffquelle angereichert wird, indem Glucose hinzugefügt wird.

9. Kulturmedium, welches durch das Verfahren gemäß einem der Ansprüche 1 bis 8 erhalten werden kann.

10. Verwendung eines Kulturmediums gemäß Anspruch 9 zur Herstellung von Mikroorganismenpopulationen.
